Europäisches Patentamt

European Patent Office

Office européen des brevets

Veröffentlichungsnummer: **0 367 251**
**A1**

# EUROPÄISCHE PATENTANMELDUNG

Anmeldenummer: **89120233.5**

Anmeldetag: **01.11.89**

Int. Cl.5 **A61B 5/00 , A61B 8/02 , A61B 5/044 , A61B 5/040**

Priorität: **04.11.88 DE 8813806 U**

Veröffentlichungstag der Anmeldung:
**09.05.90 Patentblatt 90/19**

Benannte Vertragsstaaten:
**AT BE CH DE ES FR GB GR IT LI LU NL SE**

Anmelder: **Morgenstern, Jürgen, Prof. Dr.**
**Im Heidewinkel 33**
**D-4000 Düsseldorf 12(DE)**

Erfinder: **Morgenstern, Jürgen, Prof. Dr.**
**Im Heidewinkel 33**
**D-4000 Düsseldorf 12(DE)**

Vertreter: **Plöger, Ulrich, Dipl.-Ing.**
**Benrather Schlossallee 89**
**D-4000 Düsseldorf 13(DE)**

Applikator eines Telemetrie-Systems.

Ein Applikator für ein Telemetrie-System zur Überwachung fetaler und maternaler Vitalfunktionen vor und während der Geburt besteht aus einem Sensoren (3), einen Übertragungssender (6,9) und Energiequellen (2) aufnehmendes Gehäuse (7), welches auf den Bauch der Mutter aufsetzbar ist. Für routinemäßige Untersuchungen wird das Gehäuse das als tragbares, stabförmiges Taschengerät mit Befestigungsclip (4) ausgeführt ist mit der Applikationsfläche (8) der Sensoren (3) an die Hautoberfläche angelegt.

EP 0 367 251 A1

## Applikator eines Telemetrie-Systems

Die Erfindung bezieht sich auf einen Applikator eines Telemetrie-Systems für die Überwachung fetaler und maternaler Vitalfunktionen, wie EKG, Herzschlag, Herzklappenlage und Wehen, vor und während der Geburt, wobei ein Sensoren, Übertragungssender und deren Energieversorgung aufnehmendes Gehäuse auf den Bauch der Mutter aufsetzbar ist.

Ein derartiger Applikator ist nach der DE-OS 28 26 391.4 bekannt. Er ist als eine Kapsel geringer Bauhöhe und beträchtlicher Breite ausgeführt, deren Applikationsfläche entsprechend der räumlichen Ausdehnung der Bauchrundung entsprechend konkav gestaltet ist. Diese Kapsel wird mit Hilfe eines elastischen Bandes gehalten, welche an den Enden einer sich über die Breite der Kapsel erstreckenden Traverse angreift, die ihrerseits in kraftschlüssiger Verbindung mit der Oberseite der Kapsel gehalten ist.

Von diesem Stand der Technik ausgehend liegt der Erfindung die Aufgabenstellung zugrunde, den Applikator dahingehend weiterzuentwickeln, daß er sich ohne Hilfsmittel für routinemäßige Untersuchungen eignet. Er soll insbesondere für den untersuchenden Arzt leicht verfügbar und zu handhaben sein. Vom Telemetrie-System soll dabei weiterhin Gebrauch gemacht werden.

Die genannte Aufgabenstellung wird erfindungsgemäß dadurch gelöst, daß nach dem kennzeichnenden Merkmal des Patentanspruchs 1 das Gehäuse als ein Stab von etwa zylindrischer Gestalt ausgeführt ist, und daß seine eine, als Applikationsfläche des oder der Sensoren ausgeführte Stirnseite an der Hautoberfläche zur Anlage zu bringen ist, indem der mit einer Hand zu haltende Stab aufsetzbar ist.

Ein derartiger Applikator weist gegenüber der bekannt gewesenen Kapsel beträchtliche Vorteile auf. Indem das stabförmige Gehäuse von Hand gehalten aufsetzbar ist, entfallen die sonst erforderlichen Bänder. Durch die geometrische Gestaltung als Stab verringern sich die endseitigen Stirnflächen beträchtlich, so daß insbesondere die Applikationsfläche nicht konkav ausgeführt werden muß da sie lediglich auf einen vergleichsweise kleinen Bauchabschnitt aufzusetzen ist. Weiterhin läßt sich das stabförmige Gehäuse ohne Schwierigkeiten in der Tasche mitführen, so daß die ständige Verfügbarkeit des Applikators gegeben ist. Dies erleichtert die Durchführung entsprechender Untersuchungen nicht nur im laufenden klinischen Betrieb, sondern auch bei der Ambulanz.

Im zweckmäßiger Ausgestaltung befindet sich der Übertragungssender innerhalb des Stabes in Nähe der der Applikationsfläche gegenüberliegenden Stirnseite. Diese Ausführungsform hat den Vorteil, daß man die zu den Sensoren zählenden elektronischen Bauteile in unmittelbarer Nähe der Sensorelemente anordnen kann.

Die Energieversorgung für die Sensor- und Sendereinrichtungen wird zweckmäßig durch Batterien geschaffen. Da vom jeweiligen Untersuchungsort bis zur Stelle der Anzeige- und Registriereinrichtung oft größere Abstände bestehen können, besteht ein nicht unerheblicher Leistungsbedarf. Die stabförmige Gestaltung des Gehäuses erlaubt es, diesem Leistungsbedarf entsprechend mehrere, kleinformatige Stabbatterien einzusetzen, so daß sich insbesondere bis zu sechs 1,2 V-Akkumulatoren bzw. Batterien im Stab unterbringen lassen. Sofern es sich um Akkumulatoren handelt, besteht die Möglichkeit des Anschlusses an ein Ladegerät, indem beispielsweise die der Applikationsseite gegenüberliegende Stirnseite mit Ladekontakten ausgebildet wird, die dem Stecker eines Ladegerätes entsprechen.

Die leichte Verfügbarkeit des neuen Applikators läßt sich noch dadurch steigern, daß die Stabwandung mit einem Clip versehen wird, der die Unterbringung in der Tasche eines Kleidungsstückes gestattet. Dieser Clip läßt sich in der erfindungsgemäß vorgeschlagenen Ausführungsform noch zusätzlich nutzen, indem er nicht nur in einer Stellung zu betätigen ist, sondern eine zweite Stellung einnehmen kann, in welcher er einen Einschalter der Energieversorgung schließt. Die Einschaltkontakte lassen sich auch mit den Anschlüssen eines Ladeadapters verbinden, um wiederaufladbare Batterien aufladen zu können. Der entsprechend griffig ausgeführte Clip kann von den Fingern der das Gehäuse haltenden Hand betätigt werden, so daß die Energieversorgung tatsächlich nur für den Augenblick der Untersuchung eingeschaltet sein muß.

Als Übertragungssender eignen sich insbesondere Infrarotsender, die weitgehend richtungsunabhängig ausstrahlen. Für diesen Zweck finden Infrarotstrahler Anwendung, die in die Mantelfläche des Gehäuses eingesetzt sind. Um zu vermeiden, daß die Sendesignale gestört werden, ist es zweckmäßig, diese Strahler an dem von der Applikationsfläche entfernten Mantelabschnitt des Stabes anzuordnen.

In die Applikationsfläche selbst ist vor allem die Abstrahl- und Empfangsfläche eines elektroakustischen Wandlers einbezogen. Auf diese Weise kann die bekannte Ultraschalluntersuchung mit den weiteren Untersuchungsmöglichkeiten verbunden werden. Für letztere werden vorzugsweise Widerstandsthermometer und Druckaufnehmer in die Applikationsfläche einbezogen, so daß sich Aussagen über die Temperatur und über mechanische Spannungen erzielen lassen, die für den Geburtsvorgang kennzeichnend sind. Gleichfalls zählt hierzu auch die Anordnung von Elektroden, um ein EKG aufnehmen zu können.

Die zum Sensor und Übertragungssender gehörenden Einrichtungen sind als solche bekannter Stand der Technik. Sie sind nicht nur in der gattungsgemäß zugrundegelegten DE-OS 28 26 391.4 beschrieben, sondern gleichfalls in anderen Veröffentlichungen abgehandelt. Beispielsweise befassen sich die Veröffentlichungen in "Obstetrics & Gynecology", 1979, Seiten 249 bis 254, und in "Perinatale Medizin", Sonderdruck 1978, Seiten 599 bis 602, mit Telemetriesystemen der neuerungsgemäß verwendeten Art.

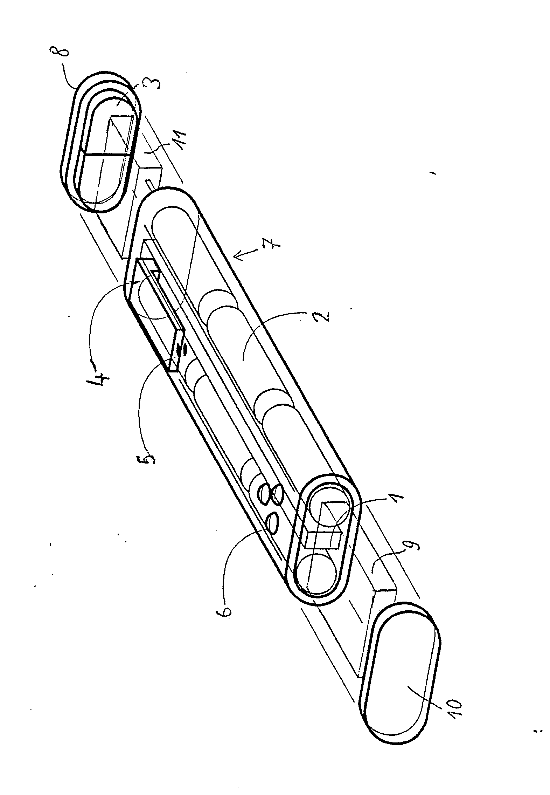

Zur näheren Veranschaulichung der Erfindung wird auf die Zeichnung Bezug genommen.

Diese Zeichnung stellt eine isometrische Darstellung des Applikators dar. Er besteht im wesentlichen aus dem Stab 7, der, wie die Stirnseite 10 erkennen läßt, von flachovalem Querschnitt ist. Im Innern des Gehäuses erkennt man sechs kleinformatige Stabbatterien, von denen eine mit dem Bezugszeichen 2 versehen ist. Zwischen den beiden Reihen der Stabbatterien 2 ist Platz für eine elektronische Einrichtung 1, die vor allem der Erzeugung der Speisespannungen und der Herstellung der Verbindungen zwischen Sensor und Übertragungssender dient. Man erkennt weiterhin einen Clip 4, der in der Normalstellung von der Mantelfläche des Gehäuses beabstandet ist, so daß er in der beschriebenen Weise bei einem Kleidungsstück festgehalten werden kann. Weiterhin erkennt man den Einschalter 5, der mit dem Clip 4 wahlweise für jede Einzeluntersuchung zu betätigen ist.

Die elektronische Einrichtung 11 für den Sensor kann insbesondere eine Ultraschall-Dopplereinrichtung sein, deren Sensor 3 bzw. Sende/Empfangs-Schwinger die Applikationsfläche 8 durchsetzt und für die Anlage an der Haut ausgebildet ist. Dabei wird in bekannter Weise von Kopplungsmitteln, wie z.B. einem Gel, Gebrauch gemacht.

Am gegenüberliegenden Ende ist das Gehäuse mit dem Übertragungssender 9 ausgeführt, der beispielsweise ein Infrarotsender ist. Die Abstrahlung findet über einen der Infrarotstrahler 6 statt. Es sind weitere Infrarotstrahler vorgesehen, so daß auch mehrere Signale übertragen werden können.

Die Teile 9 und 11 können bei entsprechender Bauweise auch in die elektronische Einrichtung 1 einbezogen werden, so daß sich die Länge des Applikators entsprechend verkürzen läßt.

Wie die Zeichnung erkennen läßt, ist der Einschalter 5 mit zwei einander gegenüberliegenden Kontakten ausgeführt, die durch den sie auch in der Öffnungsstellung überdeckenden Clip 4 geschützt sind. In der Öffnungsstellung des Clip 4 läßt sich der Applikator noch in einen Adapter einschieben, der Ladeanschlüsse aufweist, so daß über die Kontakte des Einschalters 5 wahlweise auch eine Lademöglichkeit mit den entsprechend wiederaufladbaren Batterien 2 gegeben ist. Ein derartiger Ladeadapter kann am ortsfesten Telemetrie-Empfänger angebracht sein.

## Ansprüche

1. Applikator eines Telemetrie-Systems für die Überwachung fetaler und maternaler Vitalfunktionen, wie EKG, Herzschlag, Herzklappenlage und Wehen, vor und während der Geburt, wobei ein Sensoren (3), Übertragungssender (9) und deren Energieversorgung aufnehmendes Gehäuse auf den Bauch der Mutter aufsetzbar ist,
dadurch gekennzeichnet,
daß das Gehäuse als ein Stab (7) von etwa zylindrischer Gestalt ausgeführt ist,
und daß seine eine, als Applikationsfläche (8) des oder der Sensoren (3) ausgeführte Stirnseite an der Hautoberfläche zur Anlage zu bringen ist, indem der mit einer Hand zu haltende Stab (7) aufsetzbar ist.

2. Applikator nach Anspruch 1,
dadurch gekennzeichnet,
daß sich der Übertragungssender (9) im Bereich der anderen, der Applikationsfläche (8) gegenüberliegenden Stirnseite (10) des Stabes (7) befindet.

3. Applikator nach den Ansprüchen 1 und 2,
dadurch gekennzeichnet,
daß der Stab (7) zwischen seinen beiden Stirnflächen eine Aufnahmekammer für wenigstens eine Batterie (2) zur Energieversorgung aufweist, welche Aufnahmekammer mittels einer lösbaren Kappe verschlossen ist.

4. Applikator nach den Ansprüchen 1 bis 3,
dadurch gekennzeichnet,
daß die Stabwandung mit einem Clip (4) versehen ist, der in zwei Stellungen zu bringen ist, in deren einer er einen Einschalter (5) für die Energieversorgung schließt, während er in der anderen die Unterbringung des Stabes (7) an einem Kleidungsstück gestattet.

5. Applikator nach den Ansprüchen 1 bis 4,
dadurch gekennzeichnet,
daß der Stab (7) mit wenigstens einem Infrarotsender (9) ausgeführt ist, dessen Strahler (6) an einem von der Applikationsfläche (8) entfernten Mantelabschnitt des Stabes (7) angeordnet ist.

6. Applikator nach den Ansprüchen 1 bis 5,
dadurch gekennzeichnet,
daß in die Applikationsfläche (8) die Abstrahl- und Empfangsfläche eines elektroakustischen Wandlers einbezogen ist.

7. Applikator nach den Ansprüchen 1 bis 6,
dadurch gekennzeichnet,
daß in die Applikationsfläche (8) weiterhin Elektroden, Widerstandsthermometer und Druckaufnehmer einbezogen sind.

8. Applikator nach den Ansprüchen 1 bis 7,
dadurch gekennzeichnet,
daß der Einschalter (5) zwei Kontakte aufweist, von denen der eine an der Innenseite des Clips (4) und der andere gegenüberliegend an der Stabwandung angeordnet ist, und daß beide Kontakte in der Öffnungsstellung mit den beiden Anschlüssen eines Ladeadapters in Verbindung zu bringen sind, um eine oder mehrere wiederaufladbare Batterien (2) aufladen zu können.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4413629 (B.A.DURLEY, III)<br>* Zusammenfassung; Figuren *<br>* Spalte 4, Zeile 6 - Spalte 5, Zeile 60 *<br>* Spalte 7, Zeilen 18 - 43 *<br>* Spalte 9, Zeile 43 - Spalte 10, Zeile 47 *<br>--- | 1, 3, 6, 7 | A61B5/00<br>A61B8/02<br>A61B5/0444<br>A61B5/0404 |
| A | US-A-4350164 (J.L. ALLAIN, JR.)<br>* Spalte 3, Zeile 63 - Spalte 5, Zeile 39; Figuren *<br>--- | 1-4 | |
| A | DE-A-3609913 (F.T.ERNST)<br>* Spalte 5, Zeile 36 - Seite 6, Zeile 33; Ansprüche 1-4; Figuren *<br>--- | 1-3, 5-7 | |
| A | EP-A-265694 (H.-J. UHLEMANN)<br>* Spalte 3, Zeile 18 - Seite 4, Zeile 22; Figuren *<br>--- | 1, 3, 4, 7 | |
| A,D | DE-A-2826391 (H. CZERNY U.A.)<br>* Seite 4, Zeile 12 - Seite 6, Zeile 10; Ansprüche 1-8; Figur *<br>----- | 1-3, 6, 7 | |
| | | | **RECHERCHIERTE SACHGEBIETE (Int. Cl.5)**<br><br>A61B |

Der vorliegende Recherchenbericht wurde für alle Patentansprüche erstellt

| Recherchenort | Abschlußdatum der Recherche | Prüfer |
|---|---|---|
| DEN HAAG | 16 JANUAR 1990 | RIEB K.D. |